# EUROPEAN PATENT SPECIFICATION

(11) **EP 3 344 168 B1**
(45) Date of publication and mention of the grant of the patent: **25.12.2019**
(21) Application number: 16842637.7
(22) Date of filing: 25.08.2016
(51) Int. Cl.: A61B 17/32

(54) **ENDOSCOPIC SURGICAL DEVICES AND OTHER SURGICAL DEVICES**
ENDOSKOPISCHE CHIRURGISCHE VORRICHTUNGEN UND ANDERE CHIRURGISCHE VORRICHTUNGEN
DISPOSITIFS CHIRURGICAUX ENDOSCOPIQUES ET D'AUTRES DISPOSITIFS CHIRURGICAUX

(30) Priority: 02.09.2015 US 201514843417
(43) Date of publication of application: 11.07.2018
(73) Proprietor: MicroAire Surgical Instruments, LLC, Charlottesville, VA 22911 (US)
(72) Inventor: KELLER, Douglas, Charlottesville, VA 22911 (US); VAUGHN, Shannon, Charlottesville, VA 22911 (US)
(74) Representative: Gill Jennings & Every LLP
(86) International application number: PCT/US2016/048622
(87) International publication number: WO 2017/040192

(56) References cited:
- US-A- 5 448 990
- US-A- 5 448 990
- US-A- 5 536 234
- US-A- 5 620 446
- US-A1- 2002 165 580
- US-A1- 2006 190 021
- US-A1- 2011 230 716
- US-A1- 2012 209 274
- US-A1- 2013 153 632
- US-A1- 2014 066 963
- US-A1- 2014 107 422
- US-B1- 7 628 798

## Description

### FIELD OF THE INVENTION

The present invention generally relates to surgical instruments, especially endoscopic surgical instruments, and is particularly related to surgical tools and procedures which can be used for the release of the transverse carpal ligament, as well as in other applications.

### BACKGROUND OF THE INVENTION

Examples of surgical tools which are useful for inspecting and manipulating tissues (e.g., cutting of the transverse carpal ligament) in a body cavity are described in U.S. Pat. No. 4,962,770 to Agee, U.S. Pat. No. 4,963,147 to Agee, U.S. Pat. No. 5,089,000 to Agee, U.S. Pat. No. 5,306,284 to Agee, and U.S. Pat. No. 7,918,784 to Wellborn et al. (Microaire Surgical Instruments, Inc.). Endoscopic instruments are used in well-established surgical procedures, such as for the release of the transverse carpal ligament. The devices include a cutting assembly for the dissection of the ligament and an endoscope with a camera system for visualization.

However, the endoscopic instrumentation currently in commercial usage is not completely free of any disadvantage or shortcoming and seemingly certain disadvantageous aspects are inherent to usage of endoscopic instrumentation. For example, the reason that patients undergo carpal and cubital tunnel releases is because the tissue in these regions is compressing the median and ulnar nerves, causing pain and numbness and loss of function. An endoscopic surgical device is used to cut ligaments and tissues to relieve that pressure. But, by inserting the device to perform the surgery, the device increases the pressure on the nerve, even for just the 5 minutes needed to perform the procedure, which can bring about potential additional pain and other temporary complications.

Another aspect of endoscopic instrumentation currently in widespread use is that the visualization aspect does have limitations that would seem to be unavoidable or not readily addressed practically. For example, when working with light from an endoscope and the endoscope's lens within a body cavity, moisture and light reflection on metal surfaces seemingly inevitably will complicate visibility. Some in the industry have taken a direction of trying to reduce unwanted light reflection and glare off of the metal surfaces of the instrumentation by rough-blasting the metal surfaces so that they are less like mirrors. But roughened surfaces in medical instrumentation that is to be sterilized for reuse may not be well-received, in that a non-smooth surface may be considered more uncertain to fully clean compared to a smooth surface.

Another aspect of visualization with existing endoscopic instruments is that movement (especially rotational movement) of the blade case within the patient has been needed to orient the endoscope's lens to achieve the desired image. While a user might, in the abstract, theoretically want to be able to achieve a desired visual image without moving the blade case inside the patient, with the current instrumentation, without rotation and movement of the blade case within the patient, no useable visual view is captured.

Also, a blade case of current endoscopic instrumentation occupies a certain space within the patient and when that space is needed for another surgical tool that needs that space to perform its respective function, the blade case must be removed and reinserted.

### SUMMARY OF THE INVENTION

It is an object of the invention to address the above-mentioned disadvantages and shortcomings of existing endoscopic surgical tools.

It further is an object of the invention to provide an endoscopic surgical tool relatively unsusceptible to difficulties with glare and reflected light.

It is another object of the invention to provide an endoscopic surgical tool with minimized needs to be rotated inside a patient to achieve visualization.

Also it is an object of the invention to provide an endoscopic surgical tool that can remain inside a patient in contexts where previous endoscopic surgical tools would need to be removed to make way for another surgical instrument and then reinserted when the space was again made available by removal of that other instrument.

The invention in a preferred embodiment provides a surgical device according to claim 1 and a method of constructing a surgical device according to claim 12. Further embodiments are provided in the dependent claims.

Referring to a further embodiment, the invention provides a method of constructing a surgical device (such as, e.g., an endoscopic surgical device), wherein the light-absorbing surface is constructed without performing a step of rough polishing or grit-blasting metal;

In an example not claimed, the present disclosure provides a surgical device (such as, e.g., an endoscopic surgical device), comprising: a windowed blade case comprising at least one window, the blade case having a size that accommodates passage therein of an endoscope, such as, e.g., surgical devices wherein the blade case is opaque; surgical devices wherein the at least one window is positioned at a tip of the endoscope; surgical devices wherein the endoscope is rotatable; surgical devices wherein the at least one window comprises exactly one window; surgical devices wherein the at least one window comprises two windows; surgical devices wherein the at least one window comprises three windows; surgical devices wherein the at least one window comprises a first window and a second window, wherein the first window and the second window differ as to one or both of size and shape; surgical devices wherein the at least one window is defined by a clear solid section; surgical devices comprising a first window disposed on a first side of the blade case, a second window disposed on a second side of the blade case, and a third window disposed on a bottom surface of the blade case; and other surgical devices.

In an example not claimed, the present disclosure provides a method of operating an endoscope, comprising steps of: during surgery on a patient, positioning a windowed blade case inside the patient, wherein the windowed blade case comprises at least a first window (such as, e.g., a first window that comprises open space; a first window that comprises clear plastic); through the first window, performing a certain step.

In an example not claimed, the present disclosure provides a surgical device (such as, e.g., an endoscopic device), comprising: a blade case comprising a clear first section (such as, e.g., a clear first section having a length dimension in a range of about 2.0-3.5 inches (5.08-8.89 cm); a width dimension in a range of about 0.15-0.25 inches (0.38-0.64 cm); and a thickness dimension in a range of about 0.010-0.025 inches (0.025-0.064 cm) and a light-absorbing second section; such as, e.g., surgical devices wherein the clear first section is a top section of the blade case and the light-absorbing second section is a bottom section of the blade case.

In an example not claimed, the present disclosure provides a method of previewing tissue to be cut during surgery (such as, e.g., carpal tunnel release surgery; cubital tunnel release surgery; endoscopic surgery; arthroscopic surgery; minimally invasive surgery (e.g., minimally invasive surgery where no dermal incision exceeds about 3 em; minimally invasive surgery where dermal incisions are in a range of about 1-1.5 cm; etc.), the method comprising: previewing the tissue to be cut through a window of a blade case or a cannula; such as, e.g., methods wherein the previewing step is performed without inserting and removing the blade case or cannula multiple times; methods wherein the blade case or cannula is opaque; methods wherein the window is located at a top of the blade case or cannula; methods further comprising, when previewing is performed, moving the endoscope up and down a length of the tissue to be cut; and other exemplary methods.

In an example not claimed, the present disclosure provides a surgical device (such as, e.g., an endoscopic device) comprising: a blade case comprising at least one concavity on an exterior surface thereon, such as, e.g., surgical devices wherein the at least one concavity extends lengthwise along the exterior surface of the blade case; surgical devices wherein the blade case is a flanged blade case, comprising at least one external wall that is concave; surgical devices comprising a set of external walls that are concave; surgical devices comprising an external sidewall that is concave; surgical devices comprising at least two external sidewalls that are concave; surgical devices wherein exactly two concave external sidewalls are included (such as, e.g., surgical devices further comprising a non-concave top wall and a non-concave bottom wall); surgical devices comprising an external bottom wall that is concave (such as, e.g., surgical devices wherein the non-concave top wall has a flat surface); etc.

In a further embodiment the invention provides a surgical device (such as, e.g., an endoscopic device) comprising a blade case ending in an edged tip, wherein the edged tip comprises a scraper that extends along a longitudinal axis of the blade case, the scraper being integrally a part of the blade case; such as, e.g., inventive surgical devices in which the scraper is defined by a shape selected from the group consisting of a flared shape; a protrusion; and a swept ridge; inventive surgical devices wherein no blade is included in the edged tip; inventive surgical devices wherein the edged tip is rounded; etc.

In an example not claimed, the present disclosure provides a method of using a surgical device, comprising the step of: scraping synovium during endoscopic carpal tunnel surgery, wherein the scraping is performed by the surgical device, and the surgical device is also useable for splitting muscle near fascia present during endoscopic cubital tunnel surgery; such as methods further comprising splitting muscle near fascia present during endoscopic cubital tunnel surgery, wherein the muscle-splitting is performed by the same surgical device that performs the synovium-scraping; etc.

In an example not claimed, the present disclosure provides a method of clearing tissue in endoscopic carpal tunnel surgery, comprising: scraping synovium away by bringing an edged tip of a blade case in contact with the synovium, while an endoscope is in place illuminating the synovium during the scraping step; and/or splitting muscle for cubital procedures by contacting the edged tip of the blade case with the muscle.

In an example not claimed, the present disclosure provides an endoscopic surgical device, comprising: a blade; a blade case that in an unused condition is attachable to a first handpiece; and a releasable blocking tab that moves between two positions (such as, e.g., a releasable blocking tab that is located on the blade case; a releasable blocking tab that is released by ejection of the blade case from the handpiece, wherein the released tab forms a physical block sized to prevent the blade case from being reattached to the first handpiece or attached to a second handpiece; etc.).

In an example not claimed, the present disclosure provides an endoscopic surgical device, comprising: a blade; and a usage indicator, wherein before the blade is used for a first time, the usage indicator occupies an internal position unseen by one viewing the surgical device, and upon the blade being used for the first time, the usage indicator moves to an external position that can be seen by one viewing the surgical device.

In a further embodiment, the invention provides an endoscopic surgical device, comprising a blade case, wherein the blade case is selected from the group consisting of: a blade case that occupies a volume less than 4 cm³, and/or has a cross-section not more than 0.36 cm² and/or has a height not more than 0.54 cm; a blade case, wherein the blade case occupies a volume less than 4 cm³; a blade case, wherein the blade case has a cross-section not more than 0.36 cm²; a blade case, wherein the blade case has a height not more than 0.54 cm, a blade case, wherein the blade case occupies a maximum external volume per unit length less than 0.055 in² (0.355 cm²), with a height to width ratio less than 80%, such as, e.g., inventive endoscopic surgical devices in which the blade case occupies a volume of not more than 3.41 cm³ ; inventive endoscopic surgical devices in which the blade case has a height not more than 0.54 cm; inventive surgical devices comprising a flange; inventive surgical devices comprising a blade case having a length of at least about 9 cm; etc.

### BRIEF DESCRIPTION OF THE DRAWINGS

The invention may be appreciated by reference to the figures, which are not necessarily drawn to scale:
Figs. 1-1A are perspective views of an exemplary surgical device 1 according to the invention, comprising blade 100 in, respectively, retracted position and raised position.
Figs. 2-2A are cross-sectional views of blade case 2 according to the invention useable in surgical device 1 of Fig. 1, depicting a hollow interior circular section defined by interior surface 3 of blade case 2.
Fig. 3 is a lengthwise cross-sectional view of a blade case 12 according to the invention, useable in surgical device 1 of Fig. 1. Blade case 12 has length 12L.
Fig. 3A is lengthwise cross-sectional view of blade case 12 of Fig. 3 rotated 90 degrees about the lengthwise 12L axis, showing window 11.
Fig. 3B is a close-up perspective view of windows 7, 9, 11 (Figs. 3-3A) in blade case 12 as a top view. Light cone 10 emanates from the endoscope in a straight up, standard position.
Fig. 3C is a close-up perspective view corresponding to Fig. 3B, as a side view. Light cone 10' emanates from the endoscope rotated 45 degrees to one side, to view side tissue.
Fig. 3D is a close-up perspective view corresponding to Figs. 3B-3C, as a bottom view. Light cone 10" emanates from the endoscope rotated 180 degrees to view tissue beneath the blade case 12.
Fig. 4 is a cross-sectional view of a clear-topped blade case 22 according to the invention, useable in surgical device 1 (Fig. 1) and having blade case length 22L.
Fig. 4A is a width-wise cross-sectional view of the blade case 22 of Fig. 4.
Fig. 5 is a lengthwise cross-sectional view of a clear-bottomed blade case 23 according to the invention, useable in surgical device 1 (Fig. 1) and having blade case length 23L.
Fig. 5A is a width-wise cross-sectional view of the blade case 23 of Fig. 5.
Fig. 6 is a lengthwise cross-sectional view of a flanged blade case 32 useable in surgical device 1 (Fig. 1) and having blade case length 32L.
Fig. 6A is an enlarged width-wise cross-sectional view of flanged blade case 32 (Fig. 6).
Fig. 6B corresponds to Fig. 6A, and depicts concavity depth 16D.
Fig. 7 is a cross-sectional view of ridges 21 formed into a concavity on an exterior surface of a blade case in an embodiment of the invention.
Fig. 8 is a cross-sectional view of a hooked edge 24 formed into a concavity on an exterior surface of a blade case in an embodiment of the invention.
Fig. 9 is a top view of a scraper-tipped blade case 42 useable in surgical device 1 (Fig. 1) and having blade case length 42L.
Fig. 10 is a side view of blade case 42 (Fig. 9) comprising scraper tip 40 according to the invention.
Fig. 10A is an enlarged view including scraper tip 40 from Fig. 10.
Fig. 11 is an exploded perspective view in an inventive embodiment of parts comprising main blade case body 50, windows 7, 9, 11 (Figs. 3-3A), a clear top 4 of the blade case, a clear bottom 4A of the blade case, a concave exterior surface 16 of the blade case, and a scraper tip 40.
Fig. 11A is an assembled perspective view corresponding to Fig. 11, depicting a surgical device according to an embodiment of the invention.

### DETAILED DESCRIPTION OF A PREFERRED EMBODIMENT

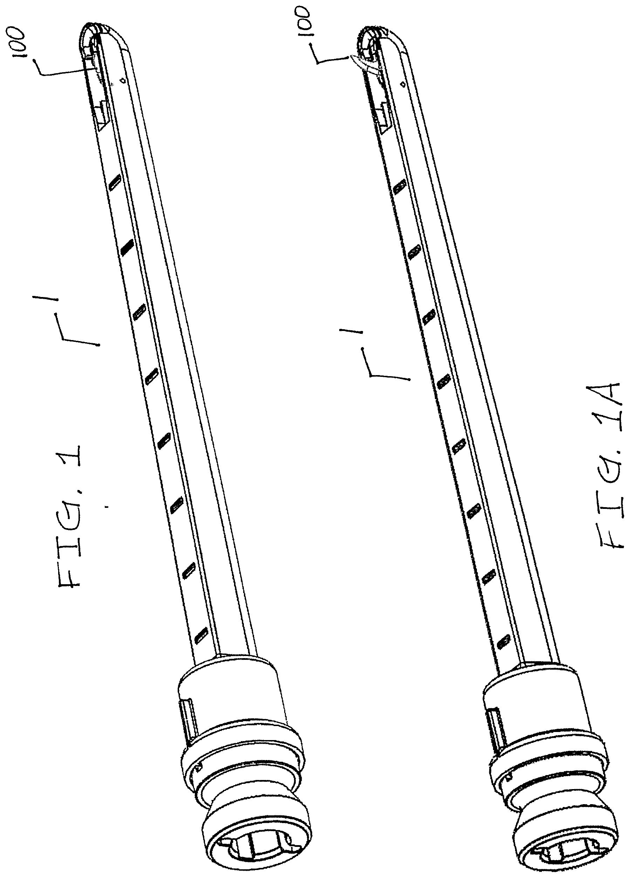

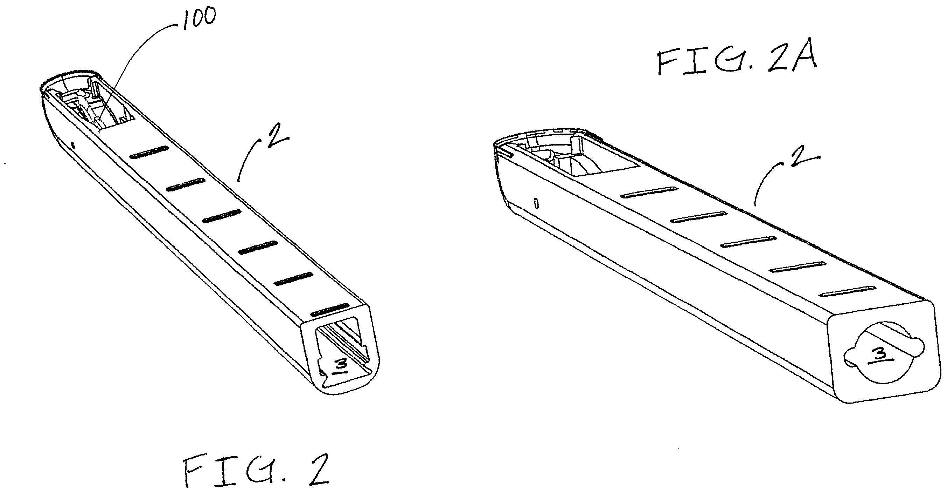

The invention provides for certain advances and improvements in surgical tools and surgical devices. In the invention, a preferred example of a surgical device is a surgical device (such as surgical device 1 in Fig. 1) comprising an endoscopic device, also referred to as "an endoscopic surgical device". The invention particularly improves upon surgical devices comprising a blade case (such as blade case 2 in Fig. 2), such as endoscopic devices comprising a blade case. Using combinations of the innovations provided herein is preferred but not mandatory.

For the interior surface 3 of the blade case 2, a light-absorbing interior surface is particularly preferred. Examples of a "light absorbing" surface are, e.g., a surface having low reflectance; a surface having low reflectivity; a surface having low albedo; a non-reflective surface; a surface having a measured light reflectance value ::::; 20% when visible light is shined thereon; etc. By a measured light reflectance value when visible light is shined thereon, we are referring to the light reflectance that is measured upon shining visible light in a range of 400-700 nm by an endoscopic instrument set to full intensity. The most preferred examples of a light absorbing surface for use in the invention are a black surface or a dark surface.

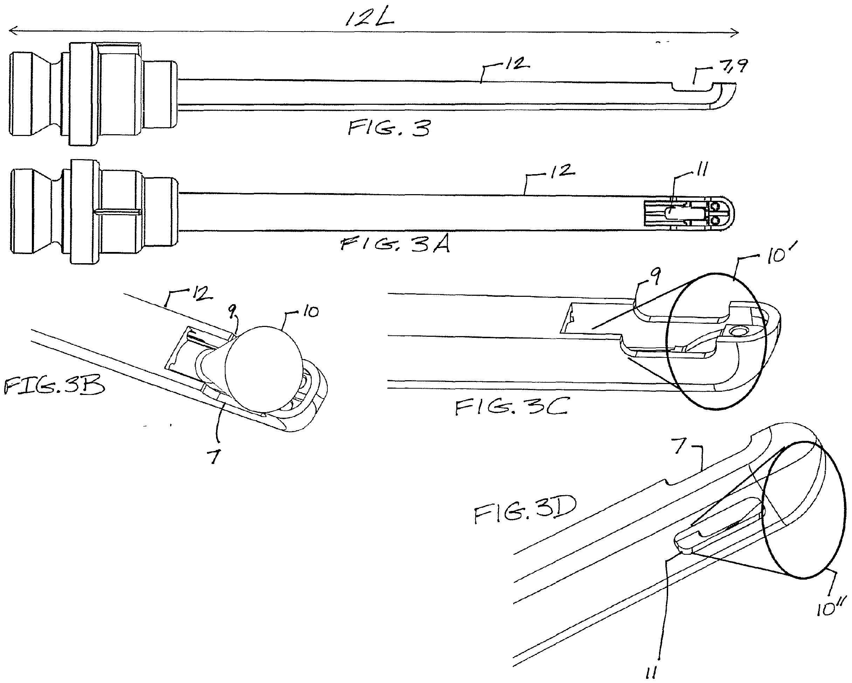

For the blade case 2, a windowed blade case (such as windowed blade case 12 in Figs. 3-3D comprising windows 7, 9, 11) is particularly preferred, with a set of three windows being a most preferred configuration.

For the set of three windows 7, 9, 11, a preferred example of the first window 11's dimensions is about 0.1-0.3 inches (0.3 cm-0.8 cm) long by 0.05-0.1 inches (0.13-0.3 cm) wide, with the second and third windows 7, 9 each respectively being about 0.2-0.5 inches (0.5-1.3 cm) long by 0.05-0.1 inches (0.13-0.3 cm) tall. In another example, the first window has a length dimension about 7mm and a width dimension in a range of about 1.5-2 mm, and the second window and third window have a length dimension about 7 mm and a width dimension in a range of about 1.5-2 mm.

Referring to Figs. 3A-3D, light cones 10, 10', 10" are depicted in a context of window 11 and windows 7, 9. Light cones 10, 10', 10" indicate the visual cone of the endoscope in three different positions.

Windows 7, 9, 11 are easily constructed such as by cutting holes through blade case 12 on the sides and bottom.

Through windows 7, 9, 11, soft tissue anatomy is viewable during surgery (with a rotating endoscope).

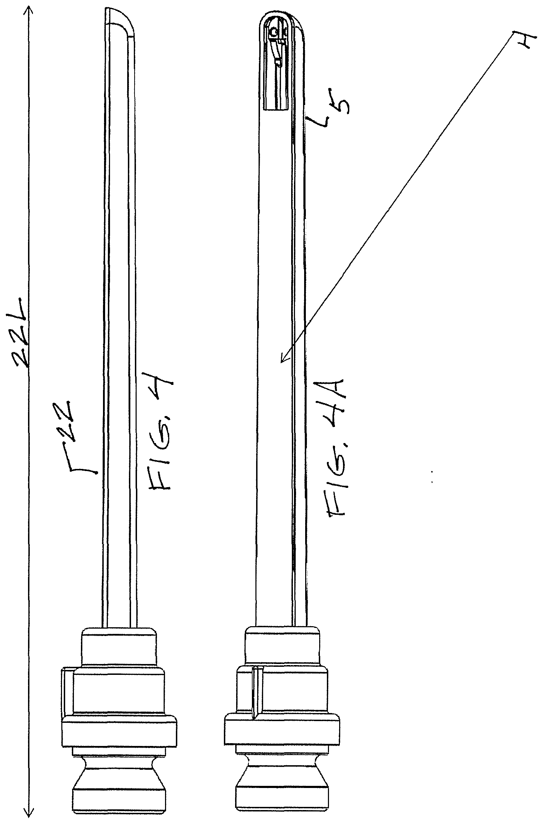

For blade case 2, a clear-topped blade case such as blade case 22 (Figs. 4-4A) is preferred. Clear top 4 in blade case 22 allows visualization via endoscope along an entire length 22L of blade case 22.

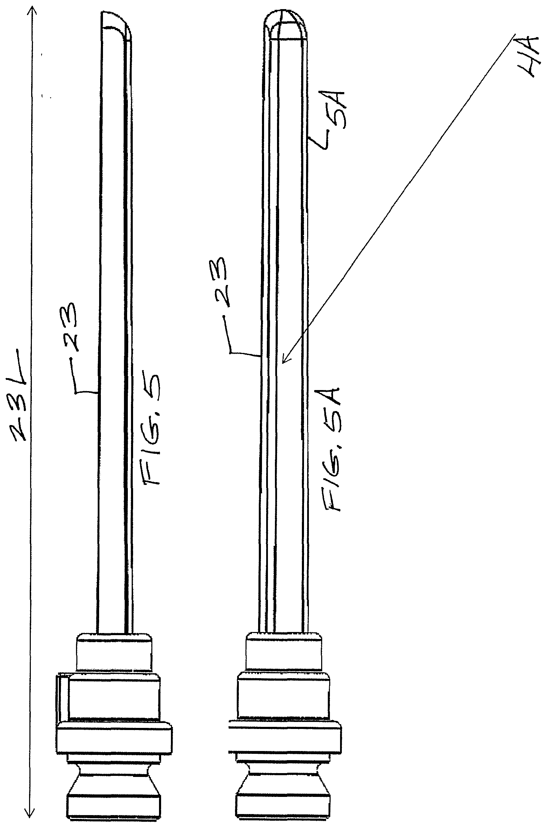

For blade case 2, a clear-bottomed blade case such as blade case 23 (Figs. 5-5A) is preferred. Clear bottom 4A allows visualization via endoscope along the entire length 23L of blade case 23.

Preferably blade case 2 is both clear-bottomed and clear-topped.

For constructing body S of blade case 22 and body SA of blade case 23, preferably a relatively stronger opaque material is used. Top 4 and bottom 4A are constructed from clear material that allows visualization via an endoscope as the endoscope is moved along lengths 22L, 23L of blade case 22, 23. The combination of materials used for top 4 and body S (and bottom 4A and body SA) is selected to maintain stiffness of the opaque design while allowing greater visualization than if top 4 (and bottom 4A) were not clear. An example of a range of thickness for clear top 4 or bottom 4A is about 0.020-0.040 inches (0.051-0.102 cm) thick.

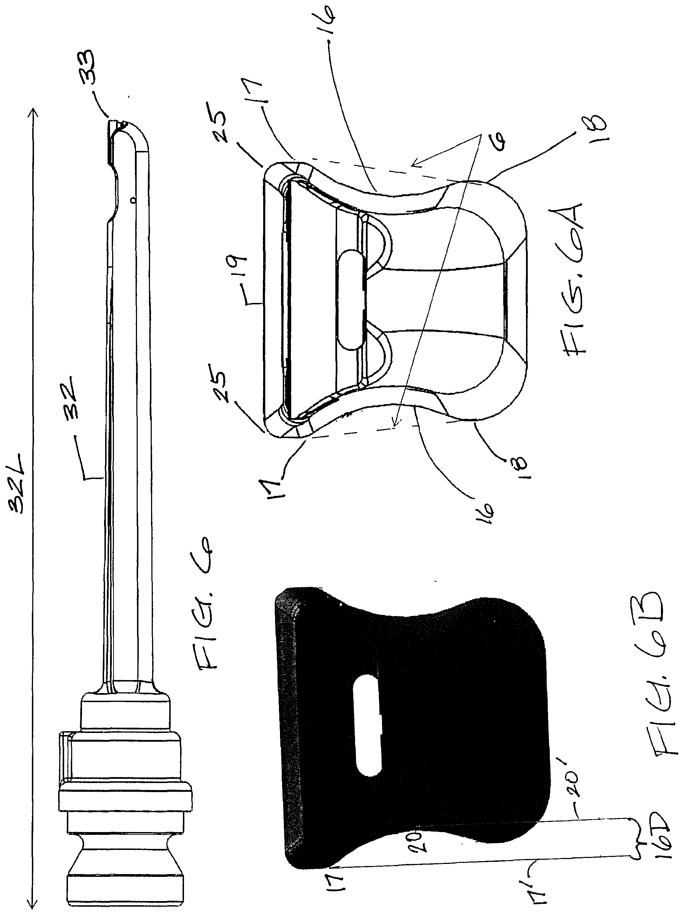

For blade case 2, a flanged blade case such as blade case 32 (Figs. 6-6B) is preferred. In Fig. 6A, the linear profile 6 of blade cases of currently-sold endoscopic instrumentation is shown as dotted lines. The invention provides for AVOIDING the linear profile 6 and instead constructing concave surfaces 16 (Fig. 6A). By forming concave surfaces 16, the cross-sectional profile in the invention approximates an hourglass shape. Concave surfaces 16 run axially along exterior of blade case 32. A concavity is defined by a concave surface 16 between non-concave surface sections 17, 18. The concavities create pockets for soft tissue to rest and hurdles for the same tissue to overcome to slide over the top 19 of blade case 32.

In Figs. 6-6B, construction of a concavity into both side surfaces of a blade case is illustrative; in other embodiments, a concavity is constructed into only one side surface of a blade case.

Concavity depth 16D (Fig. 6B) is the distance between, on the one hand, a line 17' defined by the non-concave section 17, and, on the other hand, a line 20' including the most concave point 20 of the concave surface 16 and drawn parallel to line 17'. For a blade case having length 32L of about 3.75-3.8 inches (9.53-9.65 cm), a preferred range for concavity depth 16D is about 0.025-0.050 inches (0.064-0.127 cm).

Examples of a length of a concavity are, e.g., a length equal to a full length of a blade case; a length less than a full length of a blade case. When the concavity extends less than a full length of the blade case, for the concavity length to begin at tip 33 (Fig. 6) of blade case 32 and extend backwards from the tip 33 at least about a length equal to half the blade case 32's length is preferred.

In Figs. 6-6B, construction of a concavity into a side surface of a blade case is illustrative; in other embodiments, a concavity is constructed into a bottom surface of a blade case. For example, a concave bottom surface of a blade case is considered useful particularly for working with the ulnar nerve in cubital tunnel release surgeries.

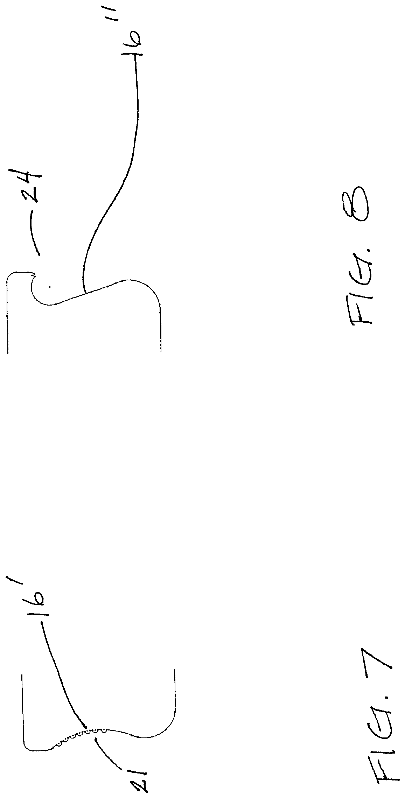

In Fig. 6A, the concave surface 16 is illustrated as smooth but it will be appreciated that the concave surface is not required to be smooth in all embodiments. For example, in some embodiments ridges 21 (Fig. 7) are formed as part of concave surface 16'. As another example, in other embodiments, surface 16" is generally-concave without being fully symmetrical, such as a concavity defined by a hooked edge 24 (Fig. 8).

As a consequence of the concave surfaces 16, flanges 25 (Fig. 6A) are formed.

An example of a shape of a tip of the blade case is a rounded edged tip, such as, e.g., a filleted, rounded-edge shape that forms a 90-180° total arc around the distal tip of the cannula, with a fillet radius of 0.001-0.010 inch (0.003-0.030 cm).

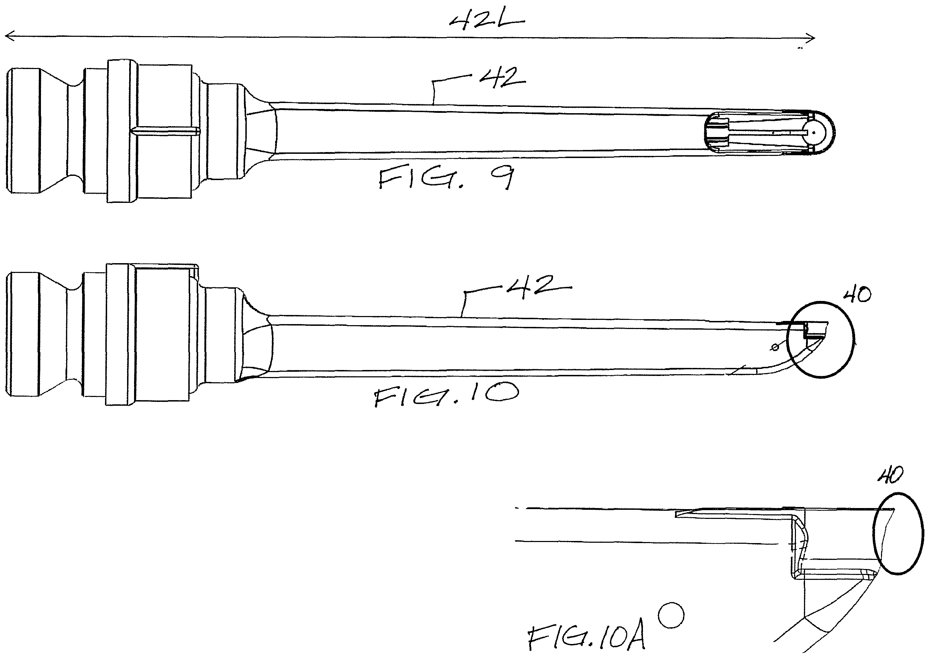

Preferably a scraper tip such as scraper tip 40 (Figs. 10-10A) is included in the blade case. Preferably scraper tip 40 is distally-flared with a sharpened edge for scraping synovium and other tissue present along the top plane of the blade case 42 during carpal tunnel surgery. As to degree of sharpness of scraper tip 40, preferably scraper tip 40 is not sharp enough to cut synovium and other biological tissue, but is sharp enough to scrape biological tissue away form the transverse ligament.

Advantageously a flared scraper tip such as scraper tip 40 also can be used to split muscle without cutting, during cubital tunnel surgery.

An endoscopic surgical instrument comprising a distally-flared scraper tip such as scraper tip 40 advantageously can remain in place within a patient when certain scraping is needed, without needing to be retracted to make room for a separate scraper to be used.

The invention may be further appreciated with reference to the following examples, without the invention being limited thereto.

### EXAMPLE 1

In this example, an inventive endoscopic surgical device according to the figures herein comprises a blade case that occupies a maximum external volume per unit length (V/L) less than 0.055 in² (0.355 cm²), with a height to width (H/W) ratio less than 80%. V/L will be appreciated to essentially reflect cross-sectional area. We refer herein to "maximum" because, for injection molded plastic parts, some amount of draft is always to be expected along the sidewalls, corresponding to reduced part size moving from the hub to the tip.

| | COMPARISON EX.* | INVENTIVE EX. |
|---|---|---|
| W | 0.25 in (0.64 cm) | 0.26 in (0.66 cm) |
| H | 0.29 in (0.74 cm) | 0.21 in (0.53 cm) |
| L | 2.75 in (6.99 cm) | at least 3.75 in (9.53 cm) |
| H/W | 112% | less than 80% |
| Max. V/L | 0.075 in² (0.484 cm²) | 0.055 in² (0.355 cm²) |

| | | |
|---|---|---|
| *Commercially sold endoscopic surgical device | | |

By contrast to the inventive example with H/W 80% or less (i.e., wider than tall), blade cases of endoscopic surgical devices currently sold have H/W 112% (i.e., taller than wide). Because this inventive example has H/W below 100%, the stiffness and strength of the blade case is reduced compared to the case with H/W 112%. To bring the stiffness and strength of the blade case to the requisite level, use of the further features of the figures herein is strongly preferred.

A reduction of 10% of the maximum V/L of commercially available endoscopic surgical devices would be considered a significant improvement by those in the industry. The context in which V/L is contemplated by those in the industry is as follows. The reason that patients undergo carpal and cubital tunnel releases is because the tissue in these regions is compressing the median and ulnar nerves, causing pain and numbness and loss of function. An endoscopic surgical device is used to cut ligaments and tissues to relieve that pressure. But, by inserting the device to perform the surgery, the device increases the pressure on the nerve, even for just the 5 minutes needed to perform the procedure, which can bring about potential additional pain and other temporary complications. The present invention's reduction of the device's V/L is highly advantageous, in that reduced V/L has an immediate and direct reduction in the pressure exerted by the device on the nerve during surgery.

### EXAMPLE 2

In this example is used a main blade case body 50 having integrally formed therein scraper tip 40 and comprising windows 7, 9 and concave walls 16. The main case body 50 in this example is opaque black, formed from a relatively-stronger material than used for clear sections 4, 4A.

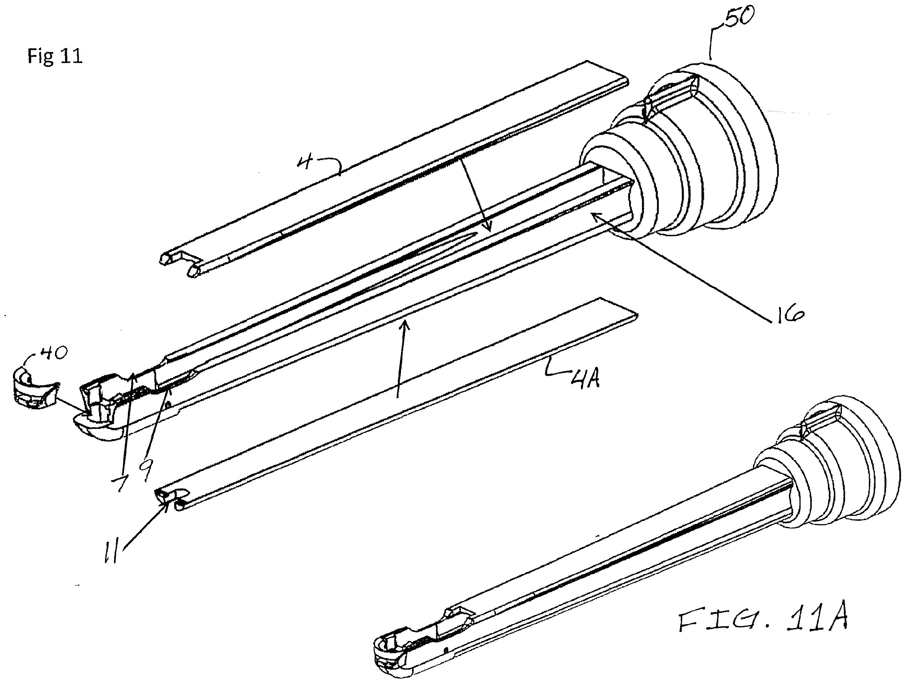

The main case body 50, clear top section 4, clear bottom section 4A (comprising window 11) are assembled into the surgical device of Fig. 11A.

While the invention has been described in terms of its preferred embodiments, those skilled in the art will recognize that the invention can be practiced with modification within the scope of the appended claims.

## Claims

1. A surgical device (1) comprising:
an interior surface (3) on which light will be shined during surgery; wherein the interior surface (3) belongs to a blade case;
**characterised in that** the blade case (2) comprises a plastic solid shape that is black or dark; and
the interior surface (3) is light-absorbing and is integral with the blade case (2) formed from a black material or a dark material.

2. The surgical device according to claim 1 wherein the height to width ratio of the blade case (2) is less than 80%.

3. The surgical device (1) according to claim 1 wherein when visible light in a range of 400-700 nanometers is shined by an endoscopic instrument set to full intensity onto the interior surface (3), a measurement of reflected light is less than 20%.

4. The surgical device (1) according to any preceding claim, wherein the surgical device (1) is an endoscopic device.

5. The surgical device (1) according to any preceding claim wherein the blade case (2) ends in an edged tip, wherein the edged tip comprises a scraper that extends along a longitudinal axis of the blade case (2), the scraper being integrally a part of the blade case (2).

6. The surgical device (1) of claim 5, wherein the scraper is defined by a shape selected from the group consisting of: a flared shape; a protrusion; and a swept ridge.

7. The surgical device (1) of claim 5, wherein no blade is included in the edged tip.

8. The surgical device (1) of claim 5, wherein the edged tip is rounded.

9. The surgical device (1) of claim 8, wherein the rounded edged tip is defined by a filleted, rounded-edge shape that forms a 90°-180° total arc around a distal tip.

10. The surgical device (1), according to any preceding claim, wherein the blade case (2) is selected from the group consisting of:
a blade case (2) that occupies a volume less than 4 cm³ and/or has a cross section not more than 0.36 cm² and/or has a height not more than 0.54 cm;
a blade case (2), wherein the blade case (2) occupies a volume less than 4 cm³;
a blade case (2), wherein the blade case (2) has a cross-section not more than 0.36 cm²;
a blade case (2), wherein the blade case (2) has a height not more than 0.54 cm;
a blade case (2), wherein the blade case (2) occupies a maximum external volume per unit length less than 0.055 in² (0.35 cm²);
a blade case (2), wherein the blade case (2) occupies a volume of not more than 3.41 cm³;
a blade case (2) having a length of at least about 3.75 inches (9.53 cm).

11. The surgical device (1) according to any preceding claim wherein the blade case is configured to accommodate a cutting blade, preferably wherein the blade is configured to be moved between a retracted position and a raised position, more preferably wherein the blade is inside the blade case when the blade is in the retracted position.

12. A method of constructing a surgical device (1), comprising:
for a surface of the surgical device (1) on which light will be shined during surgery; wherein the surface comprises a surface of a blade case,
**characterised by** constructing the surface as a light-absorbing surface, wherein the light-absorbing surface is integral with a blade case (2) formed from a black material or a dark material, wherein the constructing comprises forming a starting material into a shape that when cooled and hardened is a black plastic solid.

13. The method of claim 12, wherein the light-absorbing surface is constructed without performing a step of rough polishing or grit-blasting metal.

14. The method of claim 12 or 13 wherein the constructing comprises forming a non-layered integral part from a black material or a dark material, wherein the light-absorbing surface is a top surface of the black material or dark material.

15. The method of any of claims 12 to 14, wherein the surgical device is an endoscopic device.

16. The method of any of claims 12 to 15 wherein the height to width of the blade case is less than 80%.

## Patentansprüche

1. Chirurgische Vorrichtung (1), Folgendes umfassend:
eine innere Oberfläche (3), auf die während einer Operation Licht gestrahlt wird;
wobei die innere Oberfläche (3) zu einem Klingengehäuse gehört;
**dadurch gekennzeichnet, dass** das Klingengehäuse (2) eine feste Kunststoffform aufweist, die schwarz oder dunkel ist; und
die innere Oberfläche (3) lichtabsorbierend ist und mit dem Klingengehäuse (2), das aus einem schwarzen Material oder einem dunklen Material ausgebildet ist, einstückig ist.

2. Chirurgische Vorrichtung nach Anspruch 1, wobei das Höhen-Breiten-Verhältnis des Klingengehäuses (2) weniger als 80 % beträgt.

3. Chirurgische Vorrichtung (1) nach Anspruch 1, wobei, wenn sichtbares Licht in einem Bereich von 400-700 Nanometer durch ein endoskopisches Instrument, das auf volle Intensität auf die innere Oberfläche (3) eingestellt ist, gestrahlt wird, eine Messung von reflektiertem Licht weniger als 20 % ist.

4. Chirurgische Vorrichtung (1) nach einem der vorhergehenden Ansprüche, wobei die chirurgische Vorrichtung (1) eine endoskopische Vorrichtung ist.

5. Chirurgische Vorrichtung (1) nach einem der vorhergehenden Ansprüche, wobei das Klingengehäuse (2) in einer Kantenspitze endet, wobei die Kantenspitze einen Schaber umfasst, der sich entlang einer Längsachse des Klingengehäuses (2) erstreckt, wobei der Schaber einstückig ein Teil des Klingengehäuses (2) ist.

6. Chirurgische Vorrichtung (1) nach Anspruch 5, wobei der Schaber durch eine Form definiert ist, die aus der Gruppe ausgewählt ist, die aus Folgenden besteht:
einer aufgeweiteten Form;
einem Überstand; und
einer geschweiften Rille.

7. Chirurgische Vorrichtung (1) nach Anspruch 5, wobei keine Klinge in der Kantenspitze enthalten ist.

8. Chirurgische Vorrichtung (1) nach Anspruch 5, wobei die Kantenspitze gerundet ist.

9. Chirurgische Vorrichtung (1) nach Anspruch 8, wobei die gerundete Kantenspitze durch eine abgerundete Form mit runden Kanten definiert ist, die um eine distale Spitze einen Gesamtbogen von 90°-180° ausbildet.

10. Chirurgische Vorrichtung (1) nach einem der vorhergehenden Ansprüche, wobei das Klingengehäuse (2) aus der Gruppe, die aus Folgenden besteht, ausgewählt ist:
einem Klingengehäuse (2), das ein Volumen von weniger als 4 cm³ in Anspruch nimmt, und/oder einen Querschnitt von nicht mehr als 0,36 cm² aufweist und/oder eine Höhe von nicht mehr als 0,54 cm aufweist;
einem Klingengehäuse (2), wobei das Klingengehäuse (2) ein Volumen von weniger als 4 cm³ in Anspruch nimmt;
einem Klingengehäuse (2), wobei das Klingengehäuse (2) einen Querschnitt von nicht mehr als 0,36 cm² aufweist;
einem Klingengehäuse (2), wobei das Klingengehäuse (2) eine Höhe von nicht mehr als 0,54 cm aufweist;
einem Klingengehäuse (2), wobei das Klingengehäuse (2) ein maximales äußeres Volumen pro Längeneinheit von weniger als 0,055 sqin (0,35 cm²) in Anspruch nimmt;
einem Klingengehäuse (2), wobei das Klingengehäuse (2) ein Volumen von nicht mehr als 3,41 cm³ in Anspruch nimmt;
einem Klingengehäuse (2) mit einer Länge von wenigstens etwa 3,75 Zoll (9,53 cm).

11. Chirurgische Vorrichtung (1) nach einem der vorhergehenden Ansprüche, wobei das Klingengehäuse konfiguriert ist, um eine Schneidklinge aufzunehmen, bevorzugt wobei die Klinge konfiguriert ist, um zwischen einer eingefahrenen Position und einer angehobenen Position bewegt zu werden, besonders bevorzugt wobei sich die Klinge innerhalb des Klingengehäuses befindet, wenn sich die Klinge in der eingefahrenen Position befindet.

12. Verfahren zum Konstruieren einer chirurgischen Vorrichtung (1), umfassend:
für eine Oberfläche der chirurgischen Vorrichtung (1), auf die während der Operation Licht gestrahlt wird;
wobei die Oberfläche eine Oberfläche eines Klingengehäuses umfasst, **gekennzeichnet durch** Konstruieren der Oberfläche als eine lichtabsorbierende Oberfläche, wobei die lichtabsorbierende Oberfläche einstückig mit einem Klingengehäuse (2) ist, das aus einem schwarzen Material oder einem dunklen Material ausgebildet ist, wobei das Konstruieren ein Formen eines Ausgangsmaterials in eine Form umfasst, die, wenn sie abgekühlt und gehärtet wird, ein schwarzer Kunststofffeststoff ist.

13. Verfahren nach Anspruch 12, wobei die lichtabsorbierende Oberfläche ohne ein Ausführen eines Schrittes des Grobpolierens oder Abstrahlens von Metall konstruiert ist.

14. Verfahren nach Anspruch 12 oder 13, wobei das Konstruieren ein Ausbilden eines nicht geschichteten einstückigen Teils aus einem schwarzen Material oder aus einem dunklen Material umfasst, wobei die lichtabsorbierende Oberfläche eine obere Oberfläche des schwarzen Materials oder des dunklen Materials ist.

15. Verfahren nach einem der Ansprüche 12 bis 14, wobei die chirurgische Vorrichtung eine endoskopische Vorrichtung ist.

16. Verfahren nach einem der Ansprüche 12 bis 15, wobei die Höhe bis Breite des Klingengehäuses weniger als 80 % beträgt.

## Revendications

1. Dispositif chirurgical (1) comprenant :
une surface intérieure (3) sur laquelle de la lumière sera projetée pendant la chirurgie ;
dans lequel la surface intérieure (3) appartient à un boîtier de lame ;
**caractérisé en ce que** le boîtier de lame (2) comprend une forme solide en plastique noire ou sombre ; et
la surface intérieure (3) absorbe de la lumière et fait partie intégrante du boîtier de lame (2) constitué d'un matériau noir ou sombre.

2. Dispositif chirurgical selon la revendication 1, dans lequel le rapport hauteur/largeur du boîtier de lame (2) est inférieur à 80 %.

3. Dispositif chirurgical (1) selon la revendication 1, dans lequel, lorsque la lumière visible dans une plage de 400 à 700 nanomètres est projetée par un instrument endoscopique réglé sur une intensité maximale sur la surface intérieure (3), une mesure de la lumière réfléchie est inférieure à 20 %.

4. Dispositif chirurgical (1) selon l'une quelconque des revendications précédentes, dans lequel le dispositif chirurgical (1) est un dispositif endoscopique.

5. Dispositif chirurgical (1) selon l'une quelconque des revendications précédentes, dans lequel le boîtier de lame (2) se termine par une pointe bordée, dans lequel la pointe bordée comprend un racleur qui s'étend suivant un axe longitudinal du boîtier de lame (2), le racleur faisant partie intégrante du boîtier de lame (2).

6. Dispositif chirurgical (1) selon la revendication 5, dans lequel le racleur est défini par une forme choisie dans le groupe constitué par :
une forme évasée ;
une saillie ; et
une crête balayée.

7. Dispositif chirurgical (1) selon la revendication 5, dans lequel aucune lame n'est incluse dans la pointe bordée.

8. Dispositif chirurgical (1) selon la revendication 5, dans lequel la pointe bordée est arrondie.

9. Dispositif chirurgical (1) selon la revendication 8, dans lequel la pointe bordée arrondie est définie par une forme de bord arrondie et filetée qui forme un arc total de 90 ° à 180 ° autour d'une pointe distale.

10. Dispositif chirurgical (1) selon l'une quelconque des revendications précédentes, dans lequel le boîtier de lame (2) est choisi dans le groupe constitué par :
un boîtier de lame (2) qui occupe un volume inférieur à 4 cm³ et/ou comporte une section d'au plus 0,36 cm² et / ou une hauteur d'au plus 0,54 cm ;
un boîtier de lame (2), dans lequel le boîtier de lame (2) occupe un volume inférieur à 4 cm³ ;
un boîtier de lame (2), dans lequel le boîtier de lame (2) comporte une section d'au plus 0,36 cm² ;
un boîtier de lame (2), dans lequel le boîtier de lame (2) a une hauteur d'au plus 0,54 cm ;
un boîtier de lame (2), dans lequel le boîtier de lame (2) occupe un volume extérieur maximal par unité de longueur inférieure à 0,055 in² (0,35 cm²) ;
un boîtier de lame (2), dans lequel le boîtier de lame (2) occupe un volume d'au plus 3,41 cm³ ;
un boîtier de lame (2) ayant une longueur d'au moins environ 3,75 pouces (9,53 cm).

11. Dispositif chirurgical (1) selon l'une quelconque des revendications précédentes, dans lequel le boîtier de lame est conçu pour loger une lame de coupe, de préférence dans lequel la lame est conçue pour être déplacée entre une position rétractée et une position relevée, plus préférablement dans lequel la lame se trouve à l'intérieur du boîtier de lame lorsque la lame est en position rétractée.

12. Procédé de construction d'un dispositif chirurgical (1), comprenant :
pour une surface du dispositif chirurgical (1) sur laquelle de la lumière sera projetée pendant la chirurgie ; dans lequel la surface comprend une surface d'un boîtier de lame, **caractérisé par**
la construction de la surface sous la forme d'une surface absorbant la lumière, dans lequel la surface absorbant la lumière fait partie intégrante d'un boîtier de lame (2) constitué d'un matériau noir ou sombre,
dans lequel la construction comprend la formation d'un matériau de départ en une forme qui, une fois refroidie et durcie, est un solide en plastique noir.

13. Procédé selon la revendication 12,
dans lequel la surface absorbant la lumière est construite sans réaliser d'étape de polissage grossier ou de grenaillage de métal.

14. Procédé selon la revendication 12 ou 13, dans lequel la construction comprend la formation d'une partie intégrante non stratifiée à partir d'un matériau noir ou d'un matériau sombre, dans lequel la surface absorbant la lumière est une surface supérieure du matériau noir ou du matériau sombre.

15. Procédé selon l'une quelconque des revendications 12 à 14, dans lequel le dispositif chirurgical est un dispositif endoscopique.

16. Procédé selon l'une quelconque des revendications 12 à 15, dans lequel la hauteur par rapport à la largeur du boîtier de lame est inférieure à 80 %.
